# EUROPÄISCHE PATENTSCHRIFT

(11) **EP 1 297 119 B1**
(45) Veröffentlichungstag und Bekanntmachung des Hinweises auf die Patenterteilung: **10.11.2004**
(21) Anmeldenummer: 01960408.1
(22) Anmeldetag: 27.06.2001
(51) Int. Cl.: C12N 13/00, C12N 15/87, A61K 48/00

(54) **VERFAHREN ZUR EINBRINGUNG VON NUKLEINSÄUREN UND ANDEREN BIOLOGISCH AKTIVEN MOLEKÜLEN IN DEN KERN HÖHERER EUKARYONTISCHER ZELLEN MIT HILFE ELEKTRISCHEN STROMS**
METHOD FOR INTRODUCING NUCLEIC ACIDS AND OTHER BIOLOGICALLY ACTIVE MOLECULES INTO THE NUCLEUS OF HIGHER EUKARYOTIC CELLS BY MEANS OF AN ELECTRICAL CURRENT
PROCEDE PERMETTANT D'INTRODUIRE DES ACIDES NUCLEIQUES ET D'AUTRES MOLECULES ACTIVES D'UN POINT DE VUE BIOLOGIQUE, DANS LE NOYAU DE CELLULES EUCARYOTES SUPERIEURES, GRACE A UN COURANT ELECTRIQUE

(30) Priorität: 27.06.2000 DE 10031179
(43) Veröffentlichungstag der Anmeldung: 02.04.2003
(73) Patentinhaber: Amaxa GmbH, 50829 Köln (DE)
(72) Erfinder: SIEBENKOTTEN, Gregor, 50226 Frechen-Königsdorf (DE); CHRISTINE, Rainer, 50931 Köln (DE); ALTROGGE, Ludger, 50259 Pulheim (DE); GREMSE, Marion, 50829 Köln (DE); LENZ, Dietmar, 50676 Köln (DE); POPPENBORG, Sabine, 33803 Steinhagen (DE); RIEMEN, Gudula, 40764 Langenfeld (DE); ROTHMANN, Kirsten, 10117 Berlin (DE); THIEL, Corinna, 50823 Köln (DE)
(86) Internationale Anmeldenummer: PCT/EP2001/007348
(87) Internationale Veröffentlichungsnummer: WO 2002/000871

(56) Entgegenhaltungen:
- EP-A- 0 362 758
- WO-A-91/18103
- WO-A-92/06185
- WO-A-98/10515
- WO-A-99/36563
- US-A- 4 750 100
- US-A- 5 098 843
- US-A- 5 273 525
- KIM D ET AL: "ELECTROPORATION OF EXTRANEOUS PROTEINS INTO CHO CELLS: INCREASED EFFICACY BY UTILIZING CENTRIFUGAL FORCE AND MICROSECOND ELECTRICAL PULSES" EXPERIMENTAL CELL RESEARCH, SAN DIEGO, CA, US, Bd. 2, Nr. 197, Dezember 1991 (1991-12), Seiten 207-212, XP001064789 ISSN: 0014-4827
- DE CHASSEVAL R ET AL: "High level transient gene expression in human lymphoid cells by SV40 large T antigen boost" NUCLEIC ACIDS RESEARCH., Bd. 20, Nr. 2, 25. Januar 1992 (1992-01-25), Seiten 245-250, XP002200113
- EUROGENTEC: "Easyject Plus User's Manual" 10. Juli 1992 (1992-07-10) , EUROGENTEC , LIEGE XP002200115
- LURQUIN P: "Gene transfer by electroporation" MOL BIOTECHNOL. , Bd. 7, Nr. 1, Februar 1997 (1997-02), Seiten 5-35, XP001073618 in der Anmeldung erwähnt
- PLIQUETT U ET AL: "DETERMINATION OF THE ELECTRIC FIELD AND ANOMALOUS HEATING CAUSED BYEXPONENTIAL PULSES WITH ALUMINUM ELECTRODES IN ELECTROPORATION EXPERIMENTS" BIOELECTROCHEMISTRY AND BIOENERGETICS, XX, XX, Bd. 39, Nr. 1, 1. Februar 1996 (1996-02-01), Seiten 39-53, XP000603187 ISSN: 0302-4598
- SCHWACHTGEN JL ET AL: "Optimization of the transfection of human endothelial cells by electroporation" BIOTECHNIQUES, Bd. 17, Nr. 5, 1994, Seiten 882-887, XP002200114

## Beschreibung

Die Erfindung betrifft ein neues Verfahren, welches den Transport von DNA und/oder anderen biologisch aktiven Molekülen mit Hilfe elektrischen Stroms bis in den Kern höherer eukaryontischer Zellen, unabhängig von der Zellteilung und mit geringer Zellmortalität ermöglicht. Durch das erfindungsgemäße Verfahren wird die Zeit zwischen Transfektion und Analyse der Zellen verkürzt, was zu einer erheblichen Beschleunigung der Experimente führt. Es werden optimierte elektrische Impulse beschrieben, die für die Kernlokalisation von DNA und/oder anderen biologisch aktiven Molekülen verwendet werden können.

### Hintergrund

Da der Wirkungsort von eukaryontischer DNA der Zellkern ist, muss von außen zugegebene DNA in den Kern gelangen, um abgelesen zu werden. Herkömmliche Transfektionsmethoden bewirken nur einen Transport von DNA durch die Zellmembran ins Zytoplasma. Nur weil bei der Zellteilung höherer Eukaryonten die Kernhülle vorübergehend aufgelöst wird, kann die DNA passiv in den Kern gelangen, so dass von ihr kodierte Proteine exprimiert werden können. Nur sehr kleine DNA-Moleküle (Oligonukleotide) können frei durch die Poren der Kernhülle diffundieren. Zur effektiven Transfektion ruhender oder schwach teilungsaktiver Zellen müssen also Bedingungen geschaffen werden, die dazu führen, dass auch größere DNA-Moleküle in ausreichender Menge durch die geschlossene Kemmembran in den Kern gelangen. Die hier beschriebene Methode ermöglicht dies in höheren eukaryontischen Zellen.

### Stand der Technik

Es ist seit langem bekannt, dass man mit Hilfe elektrischen Stroms DNA aus einem Puffer in Zellen einbringen kann. Die bisher beschriebenen Versuchsbedingungen beschränken sich allerdings auf den Transport von DNA ins Zytoplasma höherer eukaryontischer Zellen, so dass die Expression transfizierter DNA von der Auflösung der Kernhülle während der Zellteilung abhängig bleibt. Keines der bisher bekannten Verfahren befasst sich damit, DNA elektrisch gezielt in den Kern höherer eukaryontischer Zellen zu bringen. Ein System, das auf elektrischen Kerntransport unabhängig von der Zellteilung optimiert ist, ist bisher nicht bekannt.

Die Entwicklung der Elektroporation geht auf die Beobachtung zurück, dass biologische Membranen durch die Wirkung kurzer elektrischer Impulse kurzzeitig durchlässiger werden (Neumann & Rosenheck 1972). Auer et al. beschrieben 1976 die Aufnahme von DNA in rote Blutkörperchen durch elektrischen Strom.

Der erste Bericht über Elektroporation von Zellinienzellen stammt aus dem Jahr 1982 (Neumann et al.). Mit kurzen Impulsen der Feldstärke 8 kV/cm und einer Dauer von je 5 µs, von denen meist drei in Abständen von drei Sekunden hintereinandergeschaltet wurden, wurde eine Maus-Fibroblastenzellinie transfiziert. Eine Analyse erfolgte nach 2 Wochen. Elektrischer Kerntransport wurde nicht beobachtet.

Durch Kurzschließen eines Transformators wurde durch Potter et al. (1984) ebenfalls eine Feldstärke von 8 kV/cm erzeugt und zur Transfektion von Zellinienzellen eingesetzt, allerdings war die Stromstärke auf max. 0,9 A begrenzt. Auch hier wurde kein elektrischer Kemtransport beobachtet.

Im weiteren Verlauf der Entwicklung wurden zunehmend längere Entladungen mit geringeren Spannungen eingesetzt, da sich gezeigt hatte, dass für eine optimale Porenöffnung der Zellmembran (bei einem mittleren Zelldurchmesser von 10-20 µm) eine Feldstärke von ca. 1 kV/cm ausreichend ist. So sind die meisten käuflichen Geräte für die Elektroporation höherer eukaryontischer Zellen und die mitgelieferten Protokolle auf Transfektionen bei diesen Feldstärken optimiert.

In einem Fall (Bertling et al., 1987) wurde die Kinetik der Verteilung der DNA in Zytoplasma und Kern in einer teilungsaktiven Zellinie verfolgt. Abgesehen von einer Erhöhung der DNA-Konzentration wurde hier jedoch kein Versuch unternommen, die frühe Aufnahme von DNA in den Kern zu verbessern. Es wurde insbesondere nicht untersucht, ob die elektrischen Parameter einen Einfluss haben könnten.

Seit 1986 wurden Patente angemeldet, die sich auf Elektroporation als Transfektionsverfahren beziehen. In diesen werden vor allem Gerätekonstruktionen und Impulsformen beschrieben. Keines greift das Problem des Transportes von DNA in den Kern außerhalb der Mitose auf.

US 4,750,100 der Firma Bio-Rad Laboratories, Richmond, USA (1986), beschreibt einen bestimmten Geräteaufbau, der durch eine Kondensatorenentladung maximal 3000 V bei maximal 125 A bereitstellen kann.

US 5,869,326 (Genetronics, Inc., San Diego, USA, 1996) beschreibt einen bestimmten Geräteaufbau, durch den mit Hilfe zweier getrennter Stromquellen zwei, drei oder mehrere Impulse erzeugt werden können. US 5,869,326 zeigt aber nicht, dass diese Impulse eine über den Transport von DNA ins Zytoplasma hinausgehende Wirkung haben.

US 6,008,038 und EP 0 866 123 A1 (Eppendorf-Netheler-Hinz GmbH, Hamburg, 1998) beschreiben ein Gerät, mit dem kurze Impulse von 10-500 µs und maximal 1,5 kV erzeugt werden können, geben aber wieder keinerlei Hinweis darauf, dass bestimmte Bedingungen dazu führen könnten, DNA in den Kern zu befördern.

Aus der WO 91/18103 A1 ist ein Verfahren zum Einbringen von DNA in Rattenzellen bekannt, bei dem die DNA durch einen ersten Impuls mit einer Feldstärke von 750 V/cm und einem zweiten Impuls mit einer Feldstärke von 250 V/cm in die Zellen eingebracht wird. Es werden ferner entsprechende Verfahren für Hefe- und Bakterienzellen beschrieben, bei denen für den ersten Impuls höhere Feldstärken von 7.500 V/cm bzw. 12.500 V/cm verwendet werden.

Kim et al. (1991) beschreiben ein Verfahren zur Einbringung von Proteinen in CHO-Zellen, bei dem die Elektroporation bei einer Feldstärke von 5.000 V/cm mit einer gleichzeitigen Zentrifugation kombiniert wird.

De Chasseval und de Villartay (1991) beschreiben ein Verfahren zur Transfektion von Zelllinien, die mit einem ersten Impuls von 1.000 V bei einer Zeitkonstante von 800 µsec und einem zweiten Impuls von 150 V bei einer Zeitkonstante von 40 msec durchgeführt wird.

Die bisher bekannten Verfahren ermöglichen aber nicht den effektiven Transport von DNA und/oder anderen biologisch aktiven Molekülen in den Zellkern höherer eukaryontischer Zellen bei geringer Mortalität der Zellen.

Der Erfindung liegt deshalb die Aufgabe zugrunde, ein Verfahren bereitzustellen, welches den effizienten Transport von DNA und/oder anderen biologisch aktiven Molekülen in den Zellkern höherer eukaryontischer Zellen bei geringer Zellmortalität unabhängig von der Zellteilung ermöglicht und das die Zeit zwischen Transfektion und nachfolgender Analyse der transfizierten Zellen erheblich verkürzt.

Die Aufgabe wird durch ein Verfahren der eingangs genannten Art gelöst, bei dem das Einbringen in den Kern primärer Zellen unabhängig von der Zellteilung durch einen Impuls mit einer Feldstärke von 2-10 KV/cm und einer Dauer von mindestens 10 µs und einer Stromstärke von mindestens 1A erreicht wird.

In der vorliegenden Anmeldung werden zum ersten Mal spezifische elektrische Bedingungen beschrieben, die effizient Kerntransport von DNA in primäre Zellen unabhängig von der Zellteilung vermitteln, und solche Entladungen und

Ströme, die dabei eine besonders geringe Zellmortalität bewirken. Auf geringe Zellmortalität optimierte Puffer und experimentelle Vorgehensweisen werden ebenfalls beschrieben.

### Beschreibung der Erfindung

Bei dem hier beschriebenen Verfahren werden sehr hohe Feldstärken von 2 bis 10 kV/cm und eine Stromstärke von mindestens 1A für eine Dauer von mindestens 10 µs dazu genutzt, dass DNA und/oder andere biologisch aktive Moleküle unabhängig von der Zellteilung in den Kern primärer Zellen gelangen können. Diese Feldstärken liegen weit über den für Elektroporation üblichen und weit jenseits der Feldstärken, die für effiziente Öffnung von Poren in der Zellmembran ausreichend sind (durchschnittlich 1 kV/cm laut Lurquin, 1997). Wahrscheinlich führen die verwendeten hohen Spannungen zu einer Porenbildung in den beiden Membranen der Kernhülle, oder die vorhandenen Kernporenkomplexe werden durchlässiger für Moleküle, so dass ein sehr effektiver Transport der biologisch aktiven Moleküle in den Zellkern ermöglicht wird. Der Impuls muss dabei eine Dauer von mindestens 10 µs aufweisen, um einen Kemtransporteffekt zu erzielen.

Der Begriff "biologisch aktive Moleküle" umfasst dabei Nukleinsäuren, Peptide, Proteine, Polysaccharide, Lipide oder Kombinationen oder Derivate dieser Moleküle, solange sie in der Zelle eine biologische Aktivität entfalten.

Das Einbringen von Nukleinsäuren, Peptiden, Proteinen und/oder anderen biologisch aktiven Molekülen in den Zellkern kann dabei vorzugsweise durch einen Impuls mit einer Feldstärke von 3-8 kV/cm erreicht werden, wobei der Impuls in vorteilhafter Ausgestaltung der Erfindung maximal 200 µs lang sein sollte. Bei einer Spannung von 1 -2 V über eine Zelle kommt es zur effizienten reversiblen Porenöffnung in der Zellmembran (Zimmermann et al., 1981). Das entspricht bei einem Zelldurchmesser von 10 - 20 µm durchschnittlich 1 kV/cm. Eine deutlich höhere Spannung sollte schon bei Pulsdauern von weniger als 1 ms zu einem irreversiblen Membranzusammenbruch führen (Zimmermann et al., 1981), wozu es aber bei Durchführung des erfindungsgemäßen Verfahrens nicht kommt. Besonders bevorzugt ist es, im erfindungsgemäßen Verfahren die Pulsdauer mit maximal 200 µs so kurz zu halten, dass auch bei 2-10 kV/cm, vorzugsweise 3-8 kV/cm, keine wesentlichen irreversiblen Membranschädigungen auftreten können, aber lang genug zu wählen, um dennoch einen Kerntransporteffekt zu erzielen.

In einer bevorzugten Ausführungsform des Verfahrens folgt auf den Impuls ohne Unterbrechung ein Stromfluss von 1 A bis maximal 2,5 A von 1 ms bis max. 50 ms Länge.

Die Transfektion durch elektrischen Strom beruht auf-zwei Effekten: der Elektropermeation der Zellmembran und der Elektrophorese von DNA durch die entstandenen Membranporen. Die beschriebenen Elektroporationsimpulse erfüllen unabhängig von ihrer Entstehung und ihrer genauen Form beide Bedingungen meist so, dass zu Beginn des Impulses eine Spannung vorliegt, die hoch genug ist, um Poren in der Zellmembran des jeweiligen Zelltyps zu öffnen, und der weitere Verlauf des Impulses ausreichend für DNA-Elektrophorese ist.

In bevorzugten Ausführungsformen der vorliegenden Erfindung werden zum Transport von DNA und/oder anderen biologisch aktiven Molekülen durch die Kernmembran in den Kern zu Anfang des Impulses für 10-200 µs Feldstärken von 2-10 kV/cm verwendet, wobei die anschließende Elektrophorese unter üblichen Bedingungen stattfindet.

Durch die Kürze des starken Impulses und die geringe Stärke und/oder Kürze des folgenden Stromflusses ist die Transfektion mit elektrischem Kerntransport trotz der sehr hohen Spannungen zu Beginn auf eine hohe Überlebensrate der Zellen optimiert. Dabei kann je nach Typ der primären Zellen eine Feineinstellung erfolgen. Da der kurze, sehr hohe Spannungsimpuls auch zur Elektrophorese der DNA beitragen kann, ermöglicht er bei einigen Zelltypen auch, dass der anschließende Stromfluss stark reduziert oder völlig weggelassen werden kann.

In einer bevorzugten Version wird eine mit Puffer, Zellen und Nukleinsäuren (und u. U. weiteren biologisch aktiven Molekülen) gefüllte Küvette zunächst einem kurzzeitigen Impuls (von 10-200 µs Länge) mit einer Feldstärke von 2-10 kV/cm ausgesetzt, dem dann ein Strom von maximal 2,5 A für 1 bis zu 50 ms folgt.

In einer bevorzugteren Version wird eine mit Puffer, Zellen und Nukleinsäuren (und u. U. weiteren biologisch aktiven Molekülen) gefüllte Küvette zunächst einem kurzzeitigen Impuls (von 10-100 µs Länge) mit einer Feldstärke von 3-8 kV/cm ausgesetzt, dem dann ohne Unterbrechung ein Strom von maximal 2,2 A für 1 bis zu 30 ms folgt.

Da diese Transfektionsmethode unabhängig von der Zellteilung ist, können damit neben teilungsaktiven Zellen auch ruhende oder schwach teilungsaktive primäre Zellen transfiziert werden.

Erfindungsgemäß werden primäre Zellen, wie beispielsweise periphere menschliche Blutzellen transfiziert, wobei es sich bevorzugt um T-Zellen, B-Zellen oder pluripotente Vorläuferzellen des menschlichen Blutes handelt.

Eine weitere bevorzugte Ausführungsform umfasst primäre humane Knochenmarkszellen.

Die mit dem erfindungsgemässen Verfahren transfizierten primären eukaryontischen Zellen können für diagnostische Verfahren und zur Herstellung eines Arzneimittels zur *ex vivo*-Gentherapie verwendet werden.

Bei teilungsaktiven primären Zellen und Zellinien läßt sich die Transfektionseffizienz steigern, da die DNA nicht bis zur Zellteilung im Zytoplasma verbleiben muss, wo sie degradiert werden könnte, und da auch die Zellen, die sich bis zur Analyse nicht geteilt haben, analysiert werden können.

Außerdem wird eine Analyse schon sehr kurz nach der Transfektion möglich, was zu einer erheblichen Beschleunigung der Experimente führt. Bei Transfektionsexperimenten mit Expressionsvektoren ist eine Analyse je nach Promotor und exprimiertem Protein schon ab ca, 2h nach der Transfektion möglich. Durch die sehr kurze Verweildauer transfizierter DNA im Zytoplasma ist die DNA dort außerdem kaum der Wirkung von Nukleasen ausgesetzt.

Mit elektrischem Kerntransport lassen sich in teilungsaktiven Zellen größere Mengen an DNA in den Kern befördern, als allein aufgrund von Zellteilung zu erwarten ist. Beides erhöht deutlich die Wahrscheinlichkeit einer Integration vollständiger Expressionskasetten.

Mit dem Begriff "elektrischer Kerntransport" ist der Transport von biologisch aktiven Molekülen in den Kern höherer eukaryontischer Zellen gemeint, der unabhängig von der Zellteilung ist und durch elektrischen Strom verursacht wird.

Das biologisch aktive Molekül, das in den Zellkern eingebracht werden soll, ist dabei bevorzugt eine Nukleinsäure, insbesondere DNA, oder weist vorzugsweise zumindest einen Nukleinsäure-Anteil auf.

Die Nukleinsäuren können dabei auch im Komplex oder in Verbindung mit Peptiden, Proteinen, Polysacchariden; Lipiden oder Kombinationen oder Derivaten dieser Moleküle vorliegen: Die mit der Nukleinsäure komplexierten oder verbundenen Moleküle dienen der Integration der transferierten Nukleinsäure ins Genom der Zelle, der intranukleären Lokalisation oder Retention, der Assoziation mit dem Chromatin oder der Regelung der Expression.

In einer bevorzugten Ausführungsform sind die mit der Nukleinsäure komplexierten Moleküle, die der Integration der transferierten Nukleinsäure ins Genom der Zelle dienen, ausgewählt aus der Gruppe umfassend retrovirale Integrasen, prokaryontische Transposasen, eukaryontische Transposasen, sequenzspezifische Rekombinasen, Topoisomerasen, *E*. *coli* RecA, *E*. *coli* RecE, *E*. *coli* RecT, Phage λ Red α, Phage λ Red β und Phage λ Terminase.

In einer besonders bevorzugten Ausführungsform umfassen die mit der Nukleinsäure komplexierten oder verbundenen Moleküle, die der intranukleären Retention oder der Assoziation mit dem Chromatin dienen, Domänen des EBV-Proteins EBNA-1. Zu diesen Domänen zählen die Aminosäuren 8-54 und/oder 72-84 oder 70-89 und/oder 328-365 des EBNA-1-Proteins (Marechal et al., 1999).

Ein Puffer, der sich zur Verwendung im erfindungsgemäßen Verfahren eignet, ist "Puffer 1" mit folgender Zusammensetzung: 0,42 mM Ca(NO₃)₂; 5,36 mM KCl; 0,41 mM MgSO₄; 103 mM NaCl; 23,8 mM NaHCO₃; 5,64 mM Na₂HPO₄; 11,1 mM D(+)-Glucose; 3,25 µM Glutathion; 20 mM Hepes; pH 7,3.

Zur Einbringung von Nukleinsäuren in den Zellkern eukaryontischer Zellen kann nach folgendem Protokoll vorgegangen werden: 1 x 10⁵ - 1 x 10⁷ Zellen und bis zu 10 µg DNA werden in 100 µl Puffer 1 in einer Küvette mit 2 mm Elektrodenabstand für 10 min. bei Raumtemperatur inkubiert und dann gemäß den erfindungsgemäßen Bedingungen transfiziert. Sofort danach werden die Zellen mit 400 µl Zellkulturmedium ohne Serum aus der Küvette gespült und für 10 min. bei 37°C inkubiert. Danach werden die Zellen in 37°C warmem Zellkulturmedium (mit . Serum) ausplattiert.

Elektrischer Kerntransport von Proteinen kann beispielsweise nach folgendem Protokoll erfolgen: Bis zu 10 µg Protein werden in 100 µl geeignetem Puffer gemäß den erfindungsgemäßen Bedingungen in 1 x 10⁵ - 1 x 10⁷ Zellen transfiziert. Sofort danach werden die Zellen mit 400 µl Zellkulturmedium ohne Serum aus der Küvette gespült und für 10 min. bei 37°C inkubiert. Danach werden die Zellen in 37°C warmem Zellkulturmedium (mit Serum) ausplattiert und nach bis zu 6 h Inkubation analysiert.

Geeignete Küvetten sind beispielsweise solche mit einem Elektrodenabstand von 2 mm oder 1 mm, wie etwa handelsübliche Küvetten für die Elektroporation von Prokaryonten.

**Die verwendeten Abkürzungen besitzen erfindungsgemäß die folgende Bedeutung:**

Außer den im Duden gebräuchlichen Akkürzungen wurden folgende Abkürzungen verwendet:
- FACS: Flurorescence activated cell sorting
- FCS: foetales Kälberserum
- h: Stunde
- kV: Kilovolt
- ms: Millisekunde
- µs: Mikrosekunde
- PBMC: periphere mononukleäre Blutzellen
- PE: Phycoerythrin

### Abbildungen

Die Erfindung wird weiterhin durch die folgenden Abbildungen erläutert:
Abbildung 1(a) und 1(b) zeigen die Transfektionseffizienz von T-Helferzellen in Abhängigkeit von der Feldstärke bei einem Impuls von 40µs Länge (a) und in Abhängigkeit von der Impulszeit bei 5 kV/cm (b).
Abbildung 2 (a) und (b) zeigen die Transfektionseffizienz von T-Helferzellen nach einem Impuls von 5 kV/cm für 40 µs, an den sich ohne Unterbrechung ein Stromfluss unterschiedlicher Stärke und Dauer anschliesst.
Abbildung 3 zeigt die FACScan-Analyse von mit dem H-2K^{k}-Expressionsvektor transfizierten PBMC. Die Zellen wurden nacheinander mit Digoxygenin-gekoppeltem anti-H-2K^{k}-Antikörper und Cy5-gekoppeltem anti-Digoxygenin-Antikörper, sowie zur Identifikation der T-Helferzellen mit einem Phycoerythrin (PE)-gekoppelten anti-CD4-Antikörper, inkubiert und durchflusszytometrisch analysiert. Mit Propidiumiodidfärbung wurde die Anzahl toter Zellen bestimmt (nicht gefärbter Fluoreszenzkanal FL3) (SSC= side scatter; FSC= forward scatter).
Abbildung 4 stellt eine FACScan-Analyse des elektrischen Kemtransportes in primären (teilungsaktiven) Endothelzellen aus humaner Nabelschnur (HUVEC) dar, die durch einen 70 µs-Impuls von 5 kV/cm, gefolgt von einem Stromfluss von 2,2 A für 10 ms transfiziert wurden. Die Zellen wurden nach 4 h nacheinander mit Digoxygenin-gekoppeltem anti-H-2K^{k}-Antikörper und Cy5-gekoppeltem anti-Digoxygenin-Antikörper, sowie Propidiumiodid (nicht gefärbte Fluoreszenzkanäle FL2 und FL3) inkubiert und durchflusszytometrisch (FACScan) analysiert (SSC= side scatter; FSC= forward scatter).
Abbildung 5 zeigt eine FACscan-Analyse des elektrischen Kerntransports in einer Zellinie (He-La), 3 Stunden nachdem diese durch einen 100 µs Impuls von 4 kV/cm mit einem H-2K^{k}-Expressionsvektor transfiziert wurde. Die Zellen wurden nach 4 h nacheinander mit Digoxygenin-gekoppeltem anti-H-2K^{k}-Antikörper und Cy5-gekoppeltem anti-Digoxygenin-Antikörper, sowie Propidiumiodid (nicht gefärbte Fluoreszenzkanäle FL2 und FL3) inkubiert und durchflusszytometrisch (FACScan) analysiert (SSC= side scatter; FSC= forward scatter).
Abbildung 6 zeigt den elektrischen Kemtransport eines Transkriptionsaktivatorproteins (HPV 18-E2) anhand der Analyse seiner Wirkung auf ein Reporterkonstrukt (pC18Sp1luc) in HeLa-Zellen. Die Messung erfolgte 6 Stunden, nachdem Protein und Plasmid durch einen 100 µs Impuls von 4 kV/cm in die Zellen eingebracht wurden.
Abbildung 7 zeigt zwei Diagramme durchflusszytometrischer Messungen des elektrischen Kerntransports von DNA-Lac-Repressor-Komplexen in CHO-Zellen, 5 ½ Stunden nachdem diese durch einen 70 µs Impuls von 5 kV/cm, ohne Unterbrechung gefolgt von einem Stromfluss von 2,2 A und 60 ms mit dem DNA-Protein-Komplex transfiziert wurden.
Abbildung 8 zeigt zwei Diagramme durchflusszytometrischer Analysen des elektrischen Kerntransports von Peptid-DNA-Komplexen in CHO- und K562-Zellen. Die Komplexe wurden in CHO-Zellen durch einen Impuls von 5 kV/cm für 70 µs, gefolgt von einem Stromfluss von 2,2 A für 40 ms und in K562-Zellen durch einen Impuls von 5 kV/cm für 100 µs, gefolgt von einem Stromfluss von 5 A für 10 ms eingebracht. Vier Stunden nach der Transfektion erfolgte die Analyse.

### Beispiele

Die nachstehenden Beispiele veranschaulichen die Erfindung, sind jedoch nicht begrenzend aufzufassen.

### Beispiel 1

### Elektrischer Kerntransport in Abhängigkeit von der Feldstärke und der Impulszeit

Frisch aufbereitete unstimulierte (teilungsinaktive) mononukleäre Zellen aus peripherem menschlichem Blut (PBMC) wurden mit einem Vektor, der für die schwere Kette des Maus MHC Klasse I Proteins H-2Kk kodiert, transfiziert. 1 x 10⁶ Zellen wurden mit 10 µg Vektor-DNA in Puffer 1 bei Raumtemperatur in eine Küvette mit 2 mm Elektrodenabstand gegeben und unter den angegebenen Bedingungen transfiziert. Unmittelbar danach wurden die Zellen mit 500 µl RPMI-Medium (ohne foetales Kälberserum, FCS) aus der Küvette gespült, für 10 min. bei 37 °C inkubiert und dann in eine Kulturschale mit vorgewärmtem Medium (mit FCS) überführt. Nach 5 h Inkubation wurden die Zellen nacheinander mit Digoxygenin-gekoppeltem anti-H-2Kk-Antikörper und Cy5-gekoppeltem anti-Digoxygenin-Antikörper, sowie zur Identifikation der T-Helferzellen mit einem Phycoerythrin (PE)-gekoppelten anti-CD4-Antikörper, inkubiert und durchflusszytometrisch (FACScan) analysiert. Durch Färbung mit Propidiumiodid wurde die Anzahl toter Zellen bestimmt.
Abbildung 1 zeigt die Transfektionseffizienz von T-Helferzellen in Abhängigkeit von der Feldstärke bei einem Impuls von 40 µs Länge (a) und in Abhängigkeit von der Impulszeit bei 5 kV/cm (b).

### Beispiel 2

### Erhöhung der Effizienz des elektrischen Kemtransportes durch einen an den Impuls anschlleßenden Stromfluss

Frisch aufbereitete unstimulierte PBMC wurden wie in Bsp. 1 beschrieben mit einem H-2Kk-Expressionsvektor transfiziert. Auf einen Impuls von 5 kV/cm für 40 µs folgte ohne Unterbrechung ein Stromfluss unterschiedlicher Stärke und unterschiedlicher Dauer. Nach 5 h Inkubation wurden die Zellen wie in Bsp.1 analysiert und die Transfektionseffizienz von T-Helferzellen bestimmt (Abb. 2).

### Beispiel 3

### Transfektion von PBMC

Frisch aufbereitete unstimulierte PBMC wurden wie in Bsp. 1 beschrieben mit einem H-2Kk-Expressionsvektor durch einen 40 µs-Impuls von 5 kV/cm, gefolgt von einem Stromfluss von 2,2 A für 20 ms transfiziert und wie in Bsp. 1 analysiert.

Abb. 3 zeigt die Analyse des Anteils transfizierter Zellen in der CD4-positiven und der CD4-negativen Fraktion der PBMC. 36 % der CD4+ Zellen und 19 % der CD4- Zellen exprimieren die transfizierte DNA. Von der Mortalitätsrate, die 26 % beträgt, sind drei Viertel auf die Transfektionsprozedur zurückzuführen.

### Beispiel 4

### Elektrischer Kerntransport in primären (teilungsaktiven) Endothelzellen aus humaner Nabelschnur (HUVEC)

HUVEC wurden wie in Bsp. 1 beschrieben mit einem H-2Kk-Expressionsvektor durch einen 70 µs-Impuls von 5 kV/cm, gefolgt von einem Stromfluss von 2,2 A für 10 ms transfiziert und nach 4 h nacheinander mit Digoxygenin-gekoppeltem anti-H-2Kk-Antikörper und Cy5-gekoppeltem anti-Digoxygenin-Antikörper, sowie Propidiumiodid inkubiert und durchflusszytometrisch (FACScan) analysiert.

Wie in Abbildung 4 gezeigt, exprimieren 58 % der Zellen die transfizierte DNA, bei einer Mortalität von 32 %. Wenn bei einer Teilungsdauer von 24 h in 100 % der Zellen durch Transfektion DNA ins Zytoplasma gelangt wäre und man eine Regenerationsphase nach der Transfektion unberücksichtigt läßt, könnten nach 4h in maximal 16 % der Zellen durch Auflösen der Kernhülle DNA in den Kern gelangt sein.

### Beispiel 5

### Elektrischer Kerntransport in einer Zellinie (HeLa)

Hela-Zellen wurden wie in Bsp. 4 beschrieben durch einen 100 □s Impuls von 4 kV/cm transfiziert und nach 3 h analysiert. Wie in Abbildung 5 gezeigt, exprimieren 28 % der Zellen die transfizierte DNA, bei einer Mortalität von 5,5 %. Wenn bei einer Teilungsdauer von 24h in 100 % der Zellen durch Transfektion DNA ins Zytoplasma gelangt wäre und man eine Regenerationsphase nach der Transfektion unberücksichtigt läßt, könnten nach 3h in maximal 12,5 % der Zellen durch Auflösen der Kernhülle DNA in den Kern gelangt sein.

### Beispiel 6

### Transfektionseffizienzen verschiedener unstimulierter primärer Zellen und Zellinien

1 x 10⁶ PBMC, andere primäre Zellen oder Zellinienzellen wurden mit der in Bsp. 1 beschriebenen Vorgehensweise mit verschiedenen Expressionsvektoren und mit den angegebenen Einstellungen analog zu Bsp.1 transfiziert. Bei der folgenden durchflusszytometrischen Analyse (FACScan) wurden die unterschiedlichen Subpopulationen durch spezifische Antikörper identifiziert. In nachfolgender Tabelle 1 sind die durchschnittlichen Transfektionseffizienzen angegeben. Für die Analyse der Transfektion von CD34⁺ Vorläuferzellen wurden 2,5-5 x 10⁶ PBMC transfiziert. Eine erste Analyse der Transfektionseffizienzen wurde nach 3,5 h durchgeführt, weil sich in dieser Zeitspanne keine bzw. bei Zellinien nur wenige Zellen geteilt haben. Mit einem Stern (*) markierte Werte wurden auch 3 Tage nach der Transfektion ermittelt und lagen auf der gleichen Höhe wie die 24 h-Werte.

### Beispiel 7

### Elektrischer Kerntransport von HPV18-E2 - Protein in HeLa - Zellen

Der elektrische Kemtransport von Proteinen kann beispielsweise durch die gleichzeitige Transfektion von Transkriptionsaktivator-Proteinen und Reporterkonstrukten, deren Expression durch die bindenden Aktivator-Moleküle im Zellkern angeschaltet werden kann, gezeigt werden. HeLa-Zellen wurden zu diesem Zweck mit dem pC18Sp1luc-Vektor, einem Plasmid, das neben einer Lumineszenz-Reportersequenz auch vier Bindungsstellen für den Papillomvirus-Transkriptionsaktivator HPV18-E2 vor der Promotor-Sequenz enthält, und gereinigtem HPV18-E2 - Protein transfiziert. 1 x 10⁶ Zellen wurden dabei mit 200 ng Vektor-DNA und 8 ng Protein in Puffer bei Raumtemperatur in eine Küvette gegeben und durch einen 100 µs Impuls von 4 kV/cm transfiziert. Die Zellen wurden nach 6 h Inkubation bei 37°C und 5 % CO₂ durch Messung der relativen Lumineszenz-Aktivität analysiert.

Abbildung 6 zeigt die Kotransfektion der Vektor-DNA mit dem Protein, wobei als Kontrollwert ein Ansatz ohne Protein dargestellt ist. Bei Kotransfektion mit 8 ng Protein ist eine deutliche Steigerung der Lumineszenz-Aktivität gegenüber den Kontrollen zu erkennen, was zeigt, dass der Transkriptionsaktivator in den Zellkern gelangt ist und zu einer Expression der Reporter-Sequenz geführt hat. Mit dem erfindungsgemäßen Verfahren ist also auch das Einbringen von Proteinen in den Zellkern eukaryontischer Zellen möglich.

### Beispiel 8

### Elektrischer Kerntransport von Antikörpern

Ein elektrischer Kemtransport von Antikörpern kann beispielsweise nach folgendem Ansatz erzielt werden. 1 x 10⁶ HeLa Zellen wurden mit einem Antikörper, gerichtet gegen den kernspezifischen Proteinkomplex ND10 in 100 µl Pufferlösung, bei Raumtemperatur mit einem 10 µs-Impuls von 4 kV/cm, ohne Unterbrechung gefolgt von einem Stromfluss von 5 A und 10 ms transfiziert. Die Zellen wurden unmittelbar nach der Abgabe des Pulses mit 400 µl Zellkulturmedium ohne Serum aus der Küvette gespült und für 10 min. bei 37°C inkubiert. Die Zellen wurden in 37°C warmem Zellkulturmedium (mit Serum) ausplattiert und für 5 h bei 37°C und 5 % CO₂ inkubiert. Danach wurden sie in Formaldehyd/Glutaraldehyd fixiert, mit Triton X100 permeabilisiert, mit einem FITC markiertem Antikörper, spezifisch für den anti ND10 Antikörper, inkubiert und fluoreszenzmikroskopisch analysiert.

### Beispiel 9

### Elektrischer Kerntransport von DNA-Protein-Komplexen in CHO - Zellen

Der elektrische Kerntransport von DNA-Protein-Komplexene kann beispielsweise durch die Repression der Expression von transfizierten Reporterplasmid-Repressor-Komplexen gezeigt werden. CHO-Zellen wurden zu diesem Zweck mit einem Vektor transfiziert (pSpe(LacO)₁-H2K^{k}), der zwischen Promotor und der H2K^{k}-Marker-Sequenz eine Lao-Operator-Sequenz aufweist, an die Lac-Repressor-Moleküle gebunden wurden. Gleichzeitig wurden die Zellen mit dem Vektor pMACS4.1 kotransfiziert, der für humanes CD4 kodiert und keine Lac-Operator-Sequenz enthält, so daß Lac-Repressor-Moleküle daran nicht spezifisch binden können. 1 x 10⁶ Zellen wurden dabei mit 1 µg H2K^{k}-Expressionsvektor-DNA mit LacO-Sequenz und 1 µg CD4-Expressionsvektor-DNA ohne LacO-Sequenz in Puffer bei Raumtemperatur mit 200 ng Lac-Repressor-Protein für 30 min. inkubiert, in eine Küvette gegeben, und durch einen Impuls von 5 kV/cm für 70 µs, gefolgt von einem Stromfluss von 2,2 A für 60 ms transfiziert. Die Zellen wurden nach 5,5 h Inkubation bei 37°C und 5 % CO₂ trypsinisiert, gefärbt und durchflusszytometrisch auf Expression des jeweiligen Markers analysiert. H2K^{k}-Expression wurde durch Inkubation mit Cy5-gekoppeltem anti-H-2K^{k}-Antikörper und CD4-Expression durch Inkubation mit Phycoerythrin (PE)-gekoppeltem anti-CD4-Antikörper analysiert.

Die in Abbildung 7 dargestellten Ergebnisse zweier Experimente zeigen die Expression der Marker-Sequenzen nach Transfektion der DNA-Protein-Komplexe jeweils mit und ohne gebundenem Lac-Repressor. Bei spezifisch gebundenem Lac-Repressor (H2K^{k}-Expressionsvektor mit LacO-Sequenz, Plasmid 1) ist eine deutliche Repression der H2K^{k}-Expression gegenüber der Kontrolle ohne Lac-Repressor zu erkennen, während ohne spezifische Lac-Repressor Bindung (CD4-Expressionsvektor ohne LacO-Sequenz, Plasmid 2) keine Repression der CD4-Expression erfolgte. Dies zeigt, dass der DNA-Protein-Komplex in den Zellkern gelangt ist und das Repressor-Protein zu einer Unterdrückung der Expression der Marker-Sequenz geführt hat. Mit dem erfindungsgemäßen Verfahren ist also auch das Einbringen von DNA-Protein-Komplexen in den Zellkern eukaryontischer Zellen möglich.

### Beispiel 10

### Elektrischer Kerntransport von Peptid-DNA-Komplexen

Der Kerntransport von Peptid-DNA-Komplexen kann beispielsweise durch eine Repression der Expression eines Reporter-Plasmids durch Bindung von PNA (peptide nucleic acid) an eine PNA-bindende Sequenz zwischen Promotor und Reporter-Kassette eines Reporter-Plasmids vor der Transfektion gezeigt werden. 1 µg H-2K^{k}-Expressionsvektor wurden in 10 mM Tris, 1mM EDTA mit 25 µM PNA-Peptid (niedrige Konzentration) oder 50 µM PNA-Peptid (hohe Konzentration) für 15 min, bei 65 °C inkubiert. Der Expressionsvektor wurde in zwei Varianten, mit und ohne spezifischer PNA-bindender Sequenz eingesetzt, ausserdem wurden unspezische PNA-Peptide (Peptid 1) und spezifisch bindende PNA-Peptide (Peptid 2) eingesetzt. Spezifische PNA-Bindung führt zur Maskierung einer Restriktionsschnittstelle und wurde durch eine entsprechende Restriktionsanalyse nachgewiesen. Die verwendeten PNA-Peptide hatten folgende DNA-bindende Sequenz: *NH*_{*2*}-CCTTTCTCCCTTC*-Peptid* (Peptid 1) oder *NH*_{*2*}-CTCTTCCTTTTTC*-Peptid* (Peptid 2). Der reine Peptidanteil hatte folgende Sequenz: *NH*_{*2*}-GKPTADDQHSTPPKKKRKVED-*COOH*. Bei der Transfektion von K562-Zellen wurde ein Peptidanteil mit folgender Sequenz eingesetzt: *NH*₂-OKPSSDDEATADSQHSTPPKKKRKVED-*COOH*. Nach der Inkubation wurden die Komplexe durch einem Impuls von 5 kV/cm für 70 µs, gefolgt von einem Stromfluss von 2,2 A für 40 ms in CHO-Zellen und durch einen Impuls von 5 kV/cm für 100 µs, gefolgt von einem Stromfluss von 5 A für 10 ms in K562-Zellen transfiziert. Nach einer 4 h Inkubation der Zellen bei 37°C und 5 % CO₂ wurden diese mit Cy5-gekoppeltem anti-H-2K^{k}-Antikörper gefärbt und durchflusszytometrisch auf H-2K^{k}-Expression analysiert.

Abbildung 8 zeigt die Wirkung spezifischer Bindung und unspezifischer Interaktion von PNA-Peptid mit Vektor-DNA auf die Expression eines Reporterkonstruktes und damit, dass der Peptid-DNA-Komplex in den Zellkern gelangt ist. Mit dem erfindungsgemäßen Verfahren ist also auch das Einbringen von Peptid-DNA-Komplexen in den Zellkern eukaryontischer Zellen möglich.

**TABELLE 1**

| **Zelltyp** | **Bedingungen** | **%+ nach 3,5 h** | **%+ nach 24 h** |
|---|---|---|---|
| PBMC | | | |
| CD4⁺ (T_{H}-Zellen) | 1000V 40µs/2,2A 30ms | 30 - 60 | 35 - 70* |
| CD8⁺ (T_{c}-Zellen) | 1000V 70µs/2,2A 10ms | 20 - 70 | 35 - 75* |
| CD14⁺ (Monozyten) | 1000V 70µs/1,0A 1ms | 30 - 50 | 10 - 20 |
| CD19⁺ (B-Zellen) | 800V 70µs/2,0A 30ms | 10 - 40 | 10 - 50* |
| CD34⁺ (Stammzellen) | 1000V 70µs/2,2A 10ms | 40 - 60 | 40 - 50 |

| Nabelschnur | | | |
|---|---|---|---|
| HUVEC | 800V 70µs/2,0A 10ms | 60 - 70 | 80 - 90 |

| embryonale Zellen des Huhns | | | |
|---|---|---|---|
| Neuronen | 800V 40µs/0,1A 1ms | 30 | 50 |
| Fibroblasten | 1000V 50µs/0,1A 1ms | 30 | 60 |

| Zellinien | | | |
|---|---|---|---|
| HeLa | 1000V 40µs/2,2A 1ms | > 30 | n.d. |
| CHO | 1000V 50µs/1,6A 10ms | 20 - 30 | n.d. |

### Literatur

Auer, D., Brandner, G., Bodemer, W. (1976), Dielectric breakdown of the red blood cell membrane and uptake of SV40 DNA and mammalian RNA. Naturwissenschaften, 63: 391

Bertling, W., Hunger-Bertling, K., Cline, M.J. (1987), Intranuclear uptake and persistence of biologically active DNA after electroporation of mammalian cells. J Biochem Biophys Methods Jul; 14(4):223-32.

de Chasseval, R., de Villartay, J.-P. (1992), High level transient gene expression In human lympoid alls by SV40 large T antigen boost. Nucleic Acids Research, Vol. 20, No. 2, 245-250

Kim, D., Lee, Y.L., Ransch, C.M., Bonelli, M.J. (1991), Electroporation of extraneous proteins into CHO cells: Increased efficacy by utilizing centrifugal force and microsecond electrical pulses. Experimental Cell Research 197, 207-212.

Lurquin, P.F. (1997), Gene transfer by electroporation. Mol Biotechnol Feb;7(1):5-35

Marechal, V., Dehee, A, Chikhi-Brachet, R, Piolot, T., Coppey-Moisan, M., Nicolas, J.-C. (1999), Mapping BBNA-1 domains involved in binding to metaphase chromosomes. J. Virol. 73:4385-4392

Neumann, E., Rosenheck, K. (1972), Permeability changes induced by electric impulses in vesicular membranes. J. Membrane Biol. 10: 279-290

Neumann, E., Schaefer-Ridder, M., Wang, Y., Hofschneider, P.H. (1982), Gene transfer into mouse lyoma cells by electroporation in high electric fields. EMBO J;1(7):841-5

Potter, H., Weir, L., Leder, P. (1984), Enhancer-dependent expression of human kappa immunoglobulin genes introduced into mouse pre-B lymphocytes by electroporation. Proc Natl Acad Sci U S A Nov; 81(22):7161-5

Zimmermann, U., Scheurich, P., Pilwat, G., Benz, R. (1981), Cells with manipulated functions: new perspectives for cell biology, medicine and technology. Angew. Chem. Int. Ed. Engl. 20: 325-344.

### Zitierte Patentschriften

US Patent Nr. 4,750,100, Ragsdale, C.W. (1988), Transfection high voltage controller

US Patent Nr. 5,869,326, Hofmann, G. A. (1999), Electroporation employing user-configured pulsing scheme

US Patent Nr. 6,008,038/ EP 0 866123 A1, Kroger, W., Jagdhuber, B., Ricklefs, H-J. (1999), Method and a circuit arrangement for the electropermeation of living cells

WO 91/18103 A1, Method and Device for making living cells permeable.

## Patentansprüche

1. Verfahren zur Einbringung von biologisch aktiven Molekülen in den Zellkern höherer eukaryontischer Zellen mit Hilfe elektrischen Stroms, wobei das Einbringen in den Kern primärer Zellen unabhängig von der Zelleitung durch einen Impuls mit einer Feldstärke von 2-10 kV/cm und einer Dauer von mindestens 10 µs und einer Stromstärke von mindestens 1 A erreicht wird.

2. Verfahren nach Anspruch 1, wobei die Feldstärke des Impulses 3-8 kV/cm beträgt.

3. Verfahren nach Anspruch 1 oder 2, wobei der Impuls maximal 200 µs lang ist.

4. Verfahren nach einem der Ansprüche 1 bis 3, wobei auf den Impuls ohne Unterbrechung ein Stromfluß von 1 A bis maximal 2,5 A von 1 ms bis max 50 ms Länge folgt.

5. Verfahren nach einem der Ansprüche 1 bis 4, wobei das biologisch aktive Molekül eine Nukleinsäure ist oder zumindest einen Nukleinsäure-Anteil aufweist.

6. Verfahren nach Anspruch 5, wobei die Nukleinsäure im Komplex oder in Verbindung mit Peptiden, Proteinen, Polysacchariden, Lipiden oder Kombinationen oder Derivaten dieser Moleküle vorliegt.

7. Verfahren nach Anspruch 6, wobei die mit der Nukleinsäure komplexierten oder verbundenen Moleküle der Integration der transferierten Nukleinsäure ins Genom der Zelle, der intranukleären Lokalisation oder Retention, der Assoziation mit dem Chromatin oder der Regelung der Expression dienen.

8. Verfahren nach Anspruch 6, wobei die mit der Nukleinsäure komplexierten Moleküle, die der Integration der transferierten Nukleinsaure ins Genom der Zelle dienen, ausgewählt sind aus der Gruppe umfassend retrovirale Integrasen, prokaryontische Transposasen, eukaryontische Transposasen, sequenzspezifische Rekombinasen, Topoisomera sen, *E*. *coli* RecA, *E*. *coli* RecE, *E*. *coli* RecT, Phage λ Red α, Phage λ Red β und Phage λ Terminase.

9. Verfahren nach Anspruch 6, wobei die mit der Nukleinsäure komplexierten oder verbundenen Moleküle, die der intranukleären Retention oder der Assoziation mit dem Chromatin dienen, Domänen des EBV-Proteins EBNA-1 umfassen.

10. Verfahren nach einem der Ansprüche 1-9, wobei die eukaryontischen Zellen periphere menschüche Blutzellen sind.

11. Verfahren nach einem der Anspruch 1-9, wobei die eukaryontischen Zellen pluripotente Vorlänferzellen des menschlichen Blutes sind.

12. Verfahren nach einem der Anspruch 1-9, wobei die eukaryontischen Zellen humane Knochenmarkszellen sind.

## Claims

1. A method for introducing biologically active molecules into the nucleus of higher eukaryotic cells using electric current, the introduction into the nucleus of primary cells not depending on cell division being achieved by a pulse having a field strength of 2-10 kV/cm and a duration of at least 10 µs, and a current of at least 1 A.

2. The method according to claim 1, wherein the field strength of the pulse is 3-8 kV/cm.

3. The method according to claims 1 or 2, wherein the pulse has a duration of max. 200 µs.

4. The method according to any of the claims 1 to 3, wherein the pulse is followed without interruption by a current flow of 1 A to max. 2.5 A with a duration of 1 ms to max. 50 ms.

5. The method according to any of the claims 1 to 4, wherein the biologically active molecule comprises a nucleic acid, or at least includes a nucleic acid portion.

6. The method according to claim 5, wherein the nucleic acid is present in complex or in association with peptides, proteins, polysaccharides, lipids, or combinations or derivatives of these molecules.

7. The method according to claim 6, wherein the molecules complexed or associated with the nucleic acid are used for the integration of the transferred nucleic acid in the genome of the cell, the intranuclear localization or retention, the association with the chromatin, or the regulation of the expression.

8. The method according to claim 6, wherein the molecules complexed with the nucleic acid which are used for the integration of the transferred nucleic acid in the genome of the cell are selected from the group comprising retroviral integrases, prokaryotic transposases, eukaryotic transposases, sequence-specific recombinases, topoisomerases, *E*. *coli* RecA, *E*. *coli* RecE, *E*. *coli* Rec T, phage λ Red α, phage λ Red β and phage λ terminase.

9. The method according to claim 6, wherein the molecules complexed or associated with the nucleic acid which are used for the intranuclear retention or the association with chromatin comprise domains of the EBV protein EBNA-1.

10. The method according to any of the claims 1-9, wherein the eukaryotic cells are peripheral human blood cells.

11. The method according to any of the claims 1-9, wherein the eukaryotic cells are pluripotent precursor cells of human blood.

12. The method according to any of the claims 1-9, wherein the eukaryotic cells are human bone marrow cells.

## Revendications

1. Procédé d'incorporation de molécules biologiquement actives dans le noyau cellulaire de cellules eucaryontiques supérieures à l'aide de courant électrique, l'incorporation dans le noyau de cellules primaires étant obtenue indépendamment de la division cellulaire au moyen d'une impulsion d'une force de champ de 2 à 10 kV/cm et d'une durée d'au moins 10 µs et d'une intensité de courant d'au moins 1 A.

2. Procédé selon la revendication 1, la force de champ de l'impulsion étant de 3 à 8 kV/cm.

3. Procédé selon la revendication 1 ou 2, dans lequel l'impulsion a une longueur maximale de 200 µs.

4. Procédé selon l'une quelconque des revendications 1 à 3, dans lequel l'impulsion est suivie sans interruption par un flux de courant de 1 A à 2,5 A au maximum et d'une longueur de 1 ms à 50 ms au maximum.

5. Procédé selon l'une quelconque des revendications 1 à 4, dans lequel la molécule active biologiquement est un acide nucléique ou présente au moins une proportion d'acide nucléique.

6. Procédé selon la revendication 5, dans lequel l'acide nucléique est présent dans le complexe ou en liaison avec des peptides, des protéines, des polysaccharides, des lipides ou des combinaisons ou dérivés des ces molécules.

7. Procédé selon la revendication 6, dans lequel les molécules en complexe ou liées servent à l'intégration de l'acide nucléique transféré dans le génome de la cellule, à la localisation ou à la rétention intranucléaire, à l'association avec la chromatine ou à la régulation de l'expression.

8. Procédé selon la revendication 6, les molécules en complexe avec l'acide nucléique qui servent à l'intégration de l'acide nucléique transféré dans le génome de la cellule étant sélectionnées dans le groupe comprenant les intégrases rétroviraux, les transposases procaryontiques, les transposases eucaryontiques, les recombinases spécifiques aux séquences, les topoisomérases, les *E*. *coli* RecA, *E*. *coli* RecE, *E*. *coli* RecT, phage λ Red α, phage λ Red β et le terminase de phage λ.

9. Procédé selon la revendication 6, dans lequel les molécules en complexe ou liées avec de l'acide nucléique qui servent à la rétention intranucléaire ou à l'association avec la chromatine comprennent des domaines de la protéine d'EBV EBNA-1.

10. Procédé selon l'une quelconque des revendications 1 à 9, dans lequel les cellules eucaryontiques sont des cellules sanguines humaines périphériques.

11. Procédé selon l'une quelconque des revendications 1 à 9, dans lequel les cellules eucaryontiques sont des cellules précurseuses pluripotentes du sang humain.

12. Procédé selon l'une quelconque des revendications 1 à 9, dans lequel les cellules eucaryontiques sont des cellules de moelle osseuse humaine.
